# EUROPEAN PATENT APPLICATION

(11) **EP 4 710 920 A1**
(43) Date of publication of application: **18.03.2026**
(21) Application number: 24200624.5
(22) Date of filing: 16.09.2024
(51) Int. Cl.: A61K 9/14, A61K 9/16, A61K 9/48, A61K 31/403, A61K 31/4422, A61K 31/505

(54) **PHARMACEUTICAL COMPOSITION COMPRISING COMBINATION OF AMLODIPINE, RAMIPRIL AND ROSUVASTATIN**

(71) Applicant: Adamed Pharma S.A., 05-152 Czosnow (PL)
(72) Inventor: KUZNIEWSKA, Magdalena, 01-887 Warszawa (PL); GAJDA, Maciej, 05-082 Stare Babice (PL); RUSIECKI, Rafal, 01-891 Warszawa (PL)
(74) Representative: Sitkowska, Jadwiga

(57) **Abstract**

A solid pharmaceutical composition comprising amlodipine or its pharmaceutically acceptable salt, in particular amlodipine besylate, ramipril and rosuvastatin or its pharmaceutically acceptable salt, in particular rosuvastatin calcium, in a single dosage unit, a process for its preparation and a single dosage unit comprising it. The composition finds its use in the treatment of cardiovascular diseases.

## Description

### Field of the invention

The present invention relates to a pharmaceutical composition comprising ternary combination of active ingredients, amlodipine, ramipril and rosuvastatin, in a single dosage form. The composition may be useful in the combined treatment of cardiovascular diseases, especially hypertension and hypercholesterolemia.

### Background of the invention

Treatment of cardiovascular diseases often requires administration of two antihypertensive medicaments having different mechanisms of action. Combined administration of two antihypertensive medicaments is also commonly accompanied by co-administration of anti hyperlipidemic agent to treat co-existing disorders of lipids metabolism, in particular hypercholesterolemia.

Although medicaments in combination may be administered in separate dosage forms, i.e. mono-compositions, there is still growing need to administer more than one active ingredient incorporated in one single dosage form, i.e. so called fixed-dose-combination, whether binary or ternary one. Such administration simplifies therapy and is very advantageous from the point of view of patient compliance to the treatment schemes, thus increasing the effectiveness of therapy. Also, cost of therapy may be lowered with such fixed-dose-combinations compared to the treatment with mono-compositions.

However, development of such fixed-dose-formulations poses some serious technological problems and difficulties and their preparation is a very complex task. All requirements imposed by marketing registration authorities for medicinal products must be taken into account and complied with.

As a general prerequisite, to avoid long-lasting periods of setting the blood pressure, such fixed-dose-combination must first of all have identical or similar bioavailability and release profile of each of its active ingredients compared to respective mono-compositions when administered separately. Otherwise switching from simultaneous administration of three mono-compositions to the ternary combination composition in already treated hypertension patients or starting such a therapy in new patients would involve the risk that the period of setting the blood pressure is too long and hypertension is not properly controlled because anti-hypertensive effect does not reach quickly the required level.

Also, such a preparation must ensure suitable content uniformity for all active ingredients.

Further problems are stability and purity of the formulation, both in terms of the contents of each single active ingredient, as well as in terms of the contents of active ingredients degradation products and other impurities over time.

Stability and purity for such ternary and binary combinations is of course related to the degradation stability of the active ingredient itself and its incompatibilities with excipients for single active ingredient taken separately. Each of active ingredients has its own requirements as to the preferred type of pharmaceutical formulation, excipients used to prepare such a pharmaceutical formulation, and the process conditions suitable for stability and purity. Those may vary between various active ingredients.

What's more and very important for fixed-dose combination products, various incompatibilities between the active ingredients themselves may exist and this may lead to enhanced formation of impurities and thus decrease of purity and stability of an active ingredient in a given fixed-dose-combination product.

All these issues have to be taken into account while developing combination composition.

Ramipril [chemical name (2S,3aS,6aS)-1[(S)-N-[(S)-1-carboxy-3-phenylpropyl]alanyl]-octahydrocyclopenta[b]pyrrole-2-carboxylic acid, 1-ethyl ester] belongs to the family of angiotensin converting enzyme (ACE) inhibitors and is approved for the treatment of hypertension. Ramipril can be used as a monotherapy or can be co-administered with other cardiovascular drugs substances as the individual products given concurrently in the separate unit dosage forms or as the fixed-dose combination at the same dosage level. Such approved combined treatment is inter alia the combination of ramipril and a calcium channel blocker amlodipine (as amlodipine besilate).

Instability of ramipril due to its sensitivity to various environmental factors is generally known in the art. These factors include heat, moisture, oxidation and, in the case of tablets, the compression processes used in manufacture. Main impurities resulted from decomposition are ramipril diacid or ramiprilat (European Pharmacopoeia: Impurity E - Imp. E, E-RAM) produced by hydrolysis the ester groups, and ramipril diketopiperazine (European Pharmacopoeia: Impurity D - Imp. D, D-RAM), formed by condensation. Oxidative decomposition of ramipril in the presence of air can result in undesired discoloration.

Amlodipine [chemical name 2-[(2-aminoethoxy)methyl]-4-(2-chlorophenyl)-1,4-dihydro-6-methyl-3,5-pyridinedicarboxylic acid 3-ethyl 5-methyl ester is a calcium channel blocker, which was developed to treat hypertension and coronary heart disease, and is used as its besylate salt. One of main impurities is dehydroamlodipine (Imp. D - D-AML) formed during storage or when amlodipine is subjected to oxidative or acidic conditions or undergoes photo or electrochemical degradation. Amlodipine is an easily decomposing compound. In the presence of moisture amlodipine besylate undergoes hydrolysis to produce acidic environment.

Rosuvastatin calcium (calcium (E,3R,5S)-7-[4-(4-fluorophenyl)-2-[methyl(methylsulfonyl)amino]-6-propan-2-ylpyrimidin-5-yl]-3,5-dihydroxyhept-6-enoate) belongs to the group of so-called statins, i.e. selective and competitive inhibitors of 3-hydroxy-3-methyl-glutaryl coenzyme A reductase, which inhibit the rate of the biosynthesis of cholesterol in liver through the inhibition of the enzyme, and is broadly used in the treatment of hypercholesterolemia. Rosuvastatin may undergo degradation under oxidative, photolytic, and thermal stress. Main degradation impurity is rosuvastatin lactone, called Impurity D. (Imp. D - D-RSV).

Stability of an active ingredient can be influenced by the choice of suitable excipients, and a further significant cause of decomposition is the mechanical stress associated with the manufacturing process.

Furthermore, there are incompatibilities of the above active substances with each other described in the prior art. Such incompatibilities cause formulation difficulties in developing a fixed dose composition containing more than one active substance. Different technical approaches can be taken under consideration when designing such a fixed dose product, such as physical separation to minimize interactions between active substances.

It is known from WO2010038091 that in combination compositions amlodipine easily interacts undesirably with the second active ingredient, which leads to decomposition reactions.

It is also known from WO2015022559 that fast decomposition of ramipril may occur in the presence of amlodipine.

According to WO2015022559, incompatibilities problems arise when ramipril in the form of stable granules is contacted with another active ingredient rosuvastatin. Decomposition of ramipril in granules initiated by rosuvastatin is observed as increased formation of Impurity D during storage. On the other hand, decomposition of rosuvastatin initiated by ramipril to form Impurity A is observed as well.

CN 101612403 and CN 101658675 disclose pharmaceutical compositions for treating cardiovascular diseases while improving compliance of drug administration comprising ternary fixed-dose combinations of calcium antagonist, including amlodipine, angiotensin-converting enzyme inhibitor (ACE-I), including ramipril, and a statin, including rosuvastatin. Neither stability, release features or content stability of such compositions is discussed nor specific formulation comprising simultaneously amlodipine, ramipril and rosuvastatin is described.

WO2013121233 discloses pharmaceutical fixed-dose composition containing amlodipine and ramipril and described as having improved stability, which comprises ramipril granulate, admixed with amlodipine besylate, microcrystalline cellulose and optionally a lubricant. Stability of ramipril in the granulate is reportedly achieved due to the use of superdisintegrant as a stabilizing agent and direct granulation using hydroxypropylmethylcellulose aqueous solution to obtain ramipril granulate. Stability, i.e. the level of impurities, in the presence of rosuvastatin and formulation comprising simultaneously amlodipine, ramipril and rosuvastatin were not described.

However, as reported in WO2015022559, it turned out that ramipril granulate disclosed in WO2013121233 and prepared using superdisintegrant as a stabilizing agent, does not solve incompatibility problems when said granulate is contacted with rosuvastatin. Decomposition of both ramipril in granules as well as rosuvastatin contacted with ramipril granules was detected.

To remove incompatibilities between ramipril granulate and rosuvastatin and improve stability of these active substances in combination WO2015022559 proposes special two-step way of ramipril granulation, involving two consecutive granulation operations, i.e. dry granulation by compaction followed by subsequent wet granulation of thus obtained compacts in a fluidization apparatus using hypromellose aqueous solution as a granulation liquid. WO2015022559 discloses also ternary fixed dose combination pharmaceutical formulation comprising ramipril, amlodipine besylate and rosuvastatin calcium in a single dosage unit, wherein active ingredients are separated from each other. Said formulation is a capsule filled with homogenous mixture of amlodipine compacts and ramipril granulate, onto which rosuvastatin calcium tablet separately prepared by tableting rosuvastatin compacts and Prosolv HD90 is placed. Thus, rosuvastatin is very well physically separated from other two active ingredients. The process is very complex, cumbersome, labour- and time-consuming, requiring two-step ramipril granulation, compacting amlodipine, as well as compacting and tableting rosuvastatin.

To solve the problem of stability of ramipril, WO2021162562 discloses ramipril granulate comprising intragranular polyvinyl alcohol (PVA) at the weight ratio ramipril to PVA from 1.1 to 6.5 and having pH value in the range 5.0 to 5.8, the granulate being prepared by wet granulation using aqueous solution of polyvinyl alcohol as a granulation solution. Stability is achieved due to the use of intragranular PVA in a quite high amount. Such granules also make it possible to overcome problems related to incompatibility and stability of fixed dose formulation comprising ramipril and amlodipine besylate in the same single dosage unit. A capsule filled with such ramipril granulate and amlodipine besylate powder blend is disclosed as an example of such a single dose unit. Reportedly, incompatibility of active substances is overcome by using a specific excipient with a dual function. Stability issues in the presence of rosuvastatin are neither discussed nor tested.

### Summary of the invention

The invention solves the problem of provision of a solid pharmaceutical composition comprising three active ingredients, amlodipine or its pharmaceutically acceptable salt, in particular amlodipine besylate, ramipril and rosuvastatin or its pharmaceutically acceptable slat, in particular rosuvastatin calcium, in a single dosage unit, i.e. so called fixed-dose-combination.

The invention further solves the problem of provision of said composition which can be produced in the simplest possible process, avoiding multiple and complicated steps and operations, especially steps using strong forces, like compaction or compression forces.

At the same time, the composition is provided that solves the problems of achieving adequate stabilization and purity of the composition as a whole, i.e. each of the active ingredients in the same single dosage unit. Furthermore, the composition is provided that solves the problem of compliance with requirements of equivalent or similar release profile and bioavailability of each of the active ingredients in the same single dosage unit compared to respective mono-compositions.

The above problems are solved by a fixed-dose solid pharmaceutical composition, comprising combination of amlodipine or its pharmaceutically acceptable salt, ramipril and rosuvastatin or its pharmaceutically acceptable salt, said composition comprising or consisting of a blend of a ramipril granulate comprising intragranular polyvinyl alcohol as a binder, the weight ratio of ramipril to polyvinyl alcohol being in the weight range from 1.1 to 10.0, and an extragranular phase, which is a powder blend comprising amlodipine or its pharmaceutically acceptable salt, rosuvastatin or its pharmaceutically acceptable salt, and an alkaline-environment producing organic or inorganic stabilizing agent.

The composition of the invention can be manufactured by the process of the invention, which involves preparation of ramipril granulate in a wet granulation process using polyvinyl alcohol as a binder, preparing solid blend of rosuvastatin or its pharmaceutically acceptable salt, amlodipine or its pharmaceutically acceptable salt, a filler, a disintegrant and an alkaline-environment producing organic or inorganic stabilizing agent, blending the ramipril granulate with said solid blend and a glidant. The composition may be used for filling capsules for oral administration.

Surprisingly, the composition of the invention allows to achieve stabilization of the complex combination of the active ingredients in the same single dosage unit in a simple manner without physical separation of active ingredients. The composition of the invention can be manufactured in a simple technological process, avoiding such operations like cumbersome prior art two-step process of production of ramipril granulate, and avoiding stress-involving operations like compaction or tableting of amlodipine and rosuvastatin. Also, wet granulation or compaction of amlodipine and rosuvastatin are not required and are omitted for the benefit of process simplicity and contents uniformity.

### Detailed description of the invention

The object of the invention is therefore a solid pharmaceutical composition, comprising ramipril, amlodipine or its pharmaceutically acceptable salt, and rosuvastatin or its pharmaceutically acceptable salt, said composition comprising or consisting of a blend of:
a) a ramipril granulate comprising or consisting of ramipril in the intimate mixture with intragranular polyvinyl alcohol as a binder, a filler, and a disintegrant, the weight ratio of ramipril to polyvinyl alcohol being in the range from 1.1 to 10.0; b) an extragranular phase comprising or consisting of:
   - b1) a powder blend of amlodipine or its pharmaceutically acceptable salt, rosuvastatin or its pharmaceutically acceptable salt, one or more fillers, a disintegrant, and an alkaline-environment producing organic or inorganic stabilizing agent, and
   - b2) a glidant.

A further object of the invention is a process for the preparation of the fixed-dose solid pharmaceutical composition as defined above, the process comprising:
- preparation of the ramipril granulate a) by wet granulation of a blend comprising ramipril, a filler, and disintegrant using a solution of polyvinyl alcohol as a granulation liquid, preferably aqueous solution of polyvinyl alcohol;
- preparation of the powder blend b1) comprising amlodipine or its pharmaceutically acceptable salt, rosuvastatin or its pharmaceutically acceptable salt, an alkaline-environment producing organic or inorganic stabilizing agent, a filler, and optionally one or more further excipients;
- blending ramipril granulate a) with the powder blend b1);
- adding the glidant b2) and homogenizing.

The process of the invention may further comprise filling the solid formulation thus obtained into capsules.

A further object of the invention is a pharmaceutical unit dosage form, comprising a capsule filled with the pharmaceutical composition of the invention as defined above or prepared using the process of the invention as defined in above.

It should be understood that any time when the term "amlodipine" is used therein it encompasses both amlodipine free base and its pharmaceutically acceptable salts, especially amlodipine besylate.

As used herein, by the term "fixed dose composition" it is understood that two or more active substances are contained in a single dosage form, such as a capsule.

Here particularly, ramipril and other active substances amlodipine and rosuvastatin are present in a single dosage form. The term "blend" is understood as a mixture of components.

The term "powder", according to Pharmacopoeia definitions (EU Ph 6.0, vol.I, p.738), means dry, solid and loose particles that have the ability to flow freely.

As used herein, the term "granulate" encompasses granules, pellets, spheres, spheronizates, beads and the other solid forms obtainable by granulation and/or spberonization, that can be used as such or further used for filling capsules or sachets or for compressing tablets.

It should be understood that any time when the term "rosuvastatin" is used therein it encompasses both rosuvastatin and its pharmaceutically acceptable salts, especially rosuvastatin calcium.

Preferably, the composition of the invention comprises amlodipine besylate and rosuvastatin calcium.

Ramipril granulate a) comprised in the composition of the invention is known as such from WO2021162562 and can be prepared in a manner described therein. It should be noted that according to WO2021162562 is an important feature.

According to the invention, pH of the water solution of 50 mg of the ramipril granulate sample with the concentration of 0.31 mg of Ramipril/ 1 mL of water, measured at 20°C, is in the range from 5.0 to 6.0.

The ramipril granulate comprises polyvinyl alcohol as a binder, at least one filler, and a disintegrant.

Preferably, the ramipril granulate a) comprises 0.50 to 5.00 wt.% of polyvinyl alcohol, such as 0.75, 1.75,1.25, 2.25, 2.75, 3.25, 3.75, 4.25, 4.5 and 4.77 wt.%, especially 1.50 wt.%, 80.00 to 95.00 wt.% of the filler, and 1.00 to 6.00 wt.% of a disintegrant, based on the total weight of the ramipril granulate a).

Preferably, the weight ratio of ramipril to polyvinyl alcohol in the ramipril granulate is in the range from 2.0 to 7.0, preferably about 3.3 or 6.7.

Preferably, the ramipril granulate a) does not include any other binder other than polyvinyl alcohol. It should be noted that ramipril granulate a) does not have any external coating.

It should be noted that no binders are included in the blend of extragranular powder phase b).

Suitable fillers may be selected from the group consisting of lactose; cellulose and its derivatives, such as microcrystalline cellulose; sugars and sugar alcohols; inorganic fillers such as calcium hydrogen phosphate, or the combinations thereof, and other inert substances which are approved for use in oral dosage forms. Preferably, in the granulate a) the filler is lactose monohydrate, in the amount of 80.00 to 95.00 wt.%, preferably 40.00-95.00% by weight, based on the total weight of the ramipril granulate a).

The most preferred filler is lactose, especially lactose monohydrate.

The percentage of the filler depends on the strength of ramipril in the solid composition of the invention and is adjusted accordingly. Thus, for ramipril dosage strength of 10 mg, the ramipril granulate a) comprises 83.50 wt.% of the filler especially lactose monohydrate, and for ramipril dosage strength of 5 mg, the ramipril granulate a) comprises 88.50 wt.% of the filler, especially lactose monohydrate.

Suitable disintegrants can be selected from the group consisting of, but not limited to, corn starch; pregelatinized starch: calcium or sodium carboxymethylcellulose (sodium croscarmellose, CMC-Ca, CMC-Na); microcrystalline cellulose; cross-linked polyvinylpyrrolidone (crosspovidone) type A or type B; and sodium starch glycolate.

The most preferred disintegrant is crosscarmellose sodium. Preferably, the ramipril granulate a) comprises 5.0 wt.% of crosscarmellose sodium.

Accordingly, one preferred composition of the invention comprises ramipril granulate a), consisting of ramipril in the amount of 10 mg, the binder polyvinyl alcohol at 1.50 wt.%, the filler lactose monohydrate at 83.50 wt.%, and the disintegrant crosscarmellose sodium at 5.00 wt.%; the weight ratio of ramipril to polyvinyl alcohol being 6.66.

Accordingly, another preferred composition of the invention comprises ramipril granulate a), consisting of ramipril in the amount of 5.00 mg, the binder polyvinyl alcohol at 1.50 wt.%, the filler lactose monohydrate at 88.50 wt.%, and the disintegrant crosscarmellose sodium at 5.00 wt.%; the weight ratio of ramipril to polyvinyl alcohol being 3.33.

Wet granulation can be accomplished by various means such as for example high-shear granulation or fluid-bed granulation.

Polyvinyl alcohol can be added both in the form of water or organic solution or as a dry powder altogether with the other excipients of the intragranular phase.

In one embodiment of the process of the invention the solvent used to prepare the granulating solution is water. In another embodiment of the process of the invention the solvent used to prepare the granulating liquid is an organic solvent, e.g., ethyl or isopropyl alcohol.

Techniques and apparatuses for performing wet-granulation are well known in the art.

In the wet granulation process, agglomeration of the active ingredient and excipients occurs to obtain a mass of larger granulate particles. The granulating liquid comprising the binder is fed, especially sprayed, onto the carrier particles as they are agitated in a closed container with blending tools and a chopper, usually in a high-shear mixer or fluidized bed. Dense granules are formed through the liquid and solid bridges that result in the granulate having compact structure and high bulk density. The granulating liquid can be simply poured into the mixture of powders. For improved dose uniformity and to obtain a more even granulate, the granulating liquid can be fed by spraying onto the carrier using a spray nozzle.

The granulation can be performed by high-shear granulation. In high-shear granulation, wherein dry powder of the carrier is placed in a mixing bowl usually containing an impeller, which revolves on a horizontal plane, and a chopper, which rotates either in the vertical or horizontal plane. The dry powders are mixed by the rotating impeller before the granulating liquid binder is sprayed onto the top of the bed of powder. Agitation is maintained by the rotating impeller until a predetermined optimum endpoint is reached. The granules are then usually sieved, transferred to another piece of equipment, such as for example a fluidized-bed dryer, for drying, and finally sieved.

The granulation can be also performed by fluidized bed wet granulation, wherein granulating liquid is fed by spraying onto the carrier particles powder bed that is maintained in a fluidized state such as by a flow of air injected at the base of the granulator. The advantage of the fluidized bed wet granulation is that drying can be performed within the same apparatus.

The powder blend b1) in the extragranular phase b) comprises an alkaline-environment producing organic or inorganic stabilizing agent which is present in an amount effective to stabilize the composition.

The term "alkaline-environment producing organic or inorganic stabilizing agent", generally includes inorganic metal oxides and hydroxides such as sodium oxide and hydroxide, potassium oxide and hydroxide, calcium oxide and hydroxide, magnesium oxide and hydroxide, aluminum oxide and hydroxide, and zinc oxide and hydroxide, and salts like salts of sodium, potassium, lithium, calcium, magnesium, aluminum with an acid selected, for example, from the group of phosphoric acid, carbonic acid, metasilicic acid, silicic acid, aluminic acid, aluminosilic acid, boric acid, acetic acid, propionic acid, succinic acid, glycolic acid, lactic acid, malic acid, tartaric acid, citric acid, maleic acid, phenylacetic acid, glutamic acid, benzoic acid, salicylic acid, fumaric acid, gluconic acid, and glycerophosphoric acid, and advantageously, the salts are sodium or potassium carbonate, sodium or potassium hydrogen carbonate, sodium or potassium metasilicate, sodium or potassium citrate, sodium or potassium monohydrogen citrate, sodium or potassium phosphate, sodium or potassium monohydrogen phosphate, sodium or potassium aluminate, sodium or potassium glutamate, calcium or magnesium oxide, calcium or magnesium hydroxide, calcium or magnesium carbonate, calcium or magnesium hydrogen carbonate, calcium or magnesium metasilicate, calcium or magnesium monohydrogen phosphate, calcium or magnesium phosphate, calcium or magnesium acetate, calcium or magnesium gluconate, calcium or magnesium glycerophosphate, magnesium aluminum metasilicate, aluminum phosphate, aluminum carbonate, aluminum acetate, aluminum metasilicate, and/or pharmaceutically acceptable solvates thereof, and also includes organic amines such as 1-adamantyl amine, tris(hydroxymethyl)ethylenediamine, triethanolamine, meglumine or L-arginine.

Preferably, the alkaline-environment producing organic or inorganic stabilizing agent is an inorganic stabilizing agent, especially selected from magnesium compounds, including magnesium inorganic salts. Most preferred inorganic stabilizing agent is magnesium oxide.

The amount of magnesium oxide stabilizing agent is in the range from 0.50 to 2.00 wt.% with respect to the total weight of the extragranular phase b).

Preferably, the alkaline-environment producing organic stabilizing agent is meglumine.

The powder blend b1) in the extragranular phase b) comprises the filler.

Suitable filler for powder blend b1) in the extragranular phase b) may be selected from the group consisting of lactose, such as lactose monohydrate; cellulose and its derivatives, such as microcrystalline cellulose; sugars and sugar alcohols, such as mannitol; starches, such a pregelatinized starches, such as corn starch; inorganic fillers such as calcium hydrogen phosphate, or the combinations thereof, and other inert substances which are approved for use in oral dosage forms. Advantageous filler may be selected from the group consisting of cellulose microcrystalline, pregelatinized starch, mannitol, lactose monohydrate and combinations thereof.

Preferably, powder blend b1) in the extragranular phase b) comprises the mixture of fillers such as mixture. of microcrystalline cellulose or mannitol with pregelatinised starch. Especially preferred is a mixture of microcrystalline cellulose and pregelatinised starch, such as corn starch. The amount of fillers depends on the strengths of rosuvastatin and amlodipine and is adjusted accordingly.

The powder blend b1) in the extragranular phase b) comprises the disintegrant. Preferably, the disintegrant is crosspovidone. The disintegrant, preferably crosspovidone, is used in customary amounts, such as in particular 1.75 wt.% with respect to the total weight of the extragranular phase b).

The extragranular phase b) comprises the glidant b2). Preferably, the glidant is sodium stearyl fumarate (PRUV). The glidant, preferably PRUV, is used in conventional amounts, such as in particular about 1 wt.%.

It should be noted that the extragranular phase b) does not have any external coating.

Preparation of the extragranular phase b) can be performed using conventional method of preparing powder blends. Such methods involve weighing of the components, screening and sieving and mixing using powder blending equipment conventional in the pharmaceutical industry, such as V-powder mixers or diffusion mixers (tumbler blenders) allowing aggressive yet gentle agitation.

Preferably, preparation of the extragranular phase involves:
- preparing a premix of amlodipine, such as amlodipine besylate, with part of the filler, such as preferably pregelatinised starch,
- preparing a premix of rosuvastatin, such as rosuvastatin calcium, with the alkaline-environment producing organic or inorganic stabilizing agent, such as preferably magnesium oxide, with part of the filler, such as preferably pregelatinised starch,
- combining screened premixes of amlodipine and rosuvastatin and all further components, preferably except the glidant b2).

Preferably, the blend of amlodipine and rosuvastatin and all further components is further first mixed with ramipril granulate and then the glidant b2) for final mixing.

Thus prepared final solid composition may be used as filling of capsules in the encapsulation step.

The exemplary process of preparing the solid composition of the invention and unit dosage form of the invention in the form of hard capsules is presented on Fig. 2.

### Examples

### Example 1

### Preparation of the composition of the invention

Compositions of the invention comprising rosuvastatin (RSV) as rosuvastatin calcium, amlodipine (AML) as amlodipine besylate, and ramipril (RAM) were prepared. The compositions of the invention for various dosage strengths of rosuvastatin/amlodipine/ramipril calculated as amlodipine base and free rosuvastatin, respectively, are presented in Tables 1A and 1B below.

### A. Preparation of ramipril granulate phase.

Ramipril granulate with the composition given in the Tables 1A and 1B below was prepared by high-shear granulation using polyvinyl alcohol aqueous solution as a granulating liquid.

The manufacturing process consisted of the following steps:
1. Weighing and sieving of granulate components: weigh specified amounts of intragranular components: lactose monohydrate, croscarmellose sodium, polyvinyl alcohol and ramipril into separate containers.
2. Preparation of granulation liquid: add a specified amount of a binder into a container with purified water while stirring, and stir until clear solution is obtained.
3. High-shear mixing: load and mix granulate components.
4. Granulation liquid addition: add granulation liquid into granulate components blend.
5. Wet massing: mix wet granulate with high speed for additional period of time.
6. Wet granulate sieving: pass wet granulate through a screen.
7. Fluid bed drying: load wet granules into fluid bed drier to remove water added in the granulation stage.
8. Granulate screening: pass dried granulate through a screen to calibrate the granulate.

### B. Preparation of rosuvastatin calcium/amlodipine besylate powder blend.

The manufacturing process consisted of the following steps:
1. Preparing rosuvastatin calcium powder premix: weigh specified amounts of rosuvastatin calcium, part of pregelatinised starch and magnesium oxide to the separate container and mix to obtain RSV powder (pre)mix.
2. Preparing amlodipine besylate powder premix: weigh specified amounts of amlodipine besylate part of pregelatinised starch and all further remaining components to a separate container and mix to obtain AML powder (pre)mix.
3. Screen RSV powder premix to a final container, i.e. diffusion mixer (tumble) and subsequently screen AML powder premix, and the remaining ingredients thereto and mix to obtain a blend.

### C. Preparation of final extragranular powder.

Screen sodium stearyl fumarate as a glidant and add it to the blend obtained in operation B above in the diffusion mixer and mix to obtain final extragranular powder blend.

Final extragranular powder blend is mixed with ramipril granulate, then screened and subsequently granulate - powder encapsulation is blend used to fill hard gelatin capsules.

Further compositions of the invention as presented in Tables 1C to 1J below were prepared in an analogous manner.

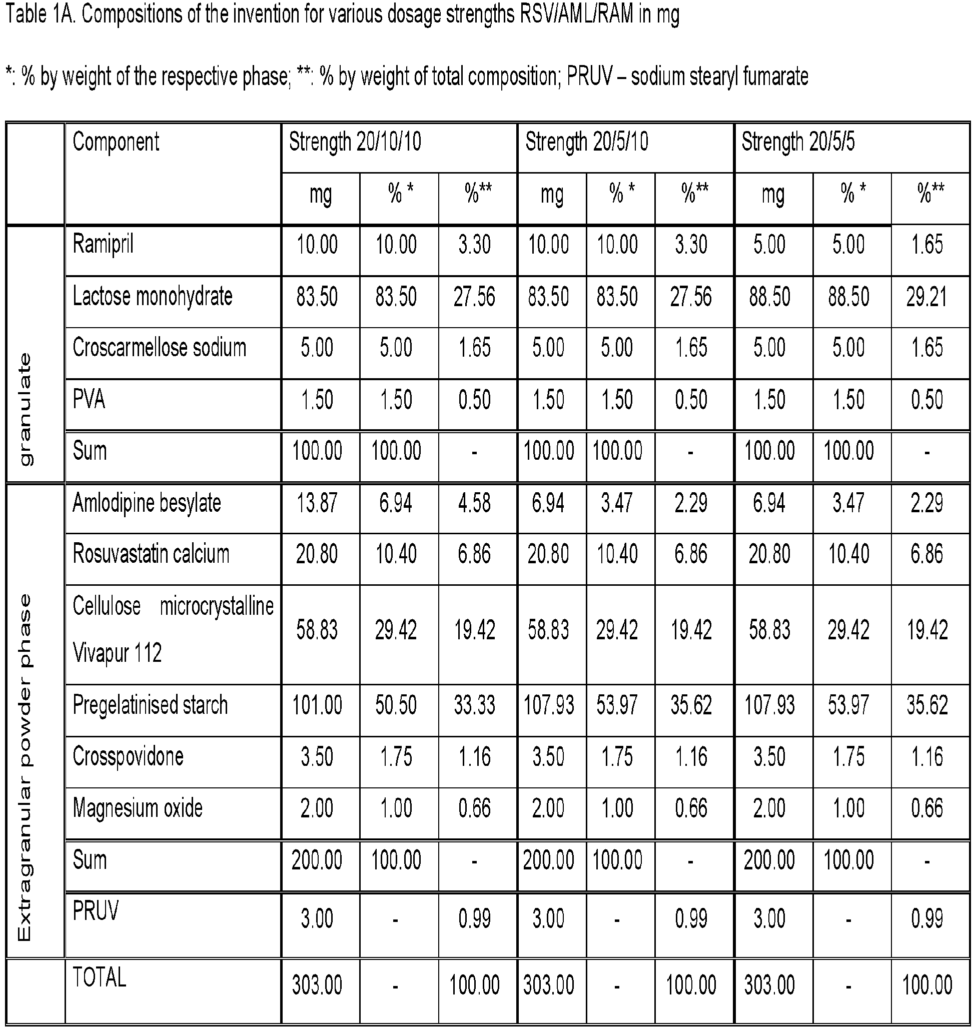

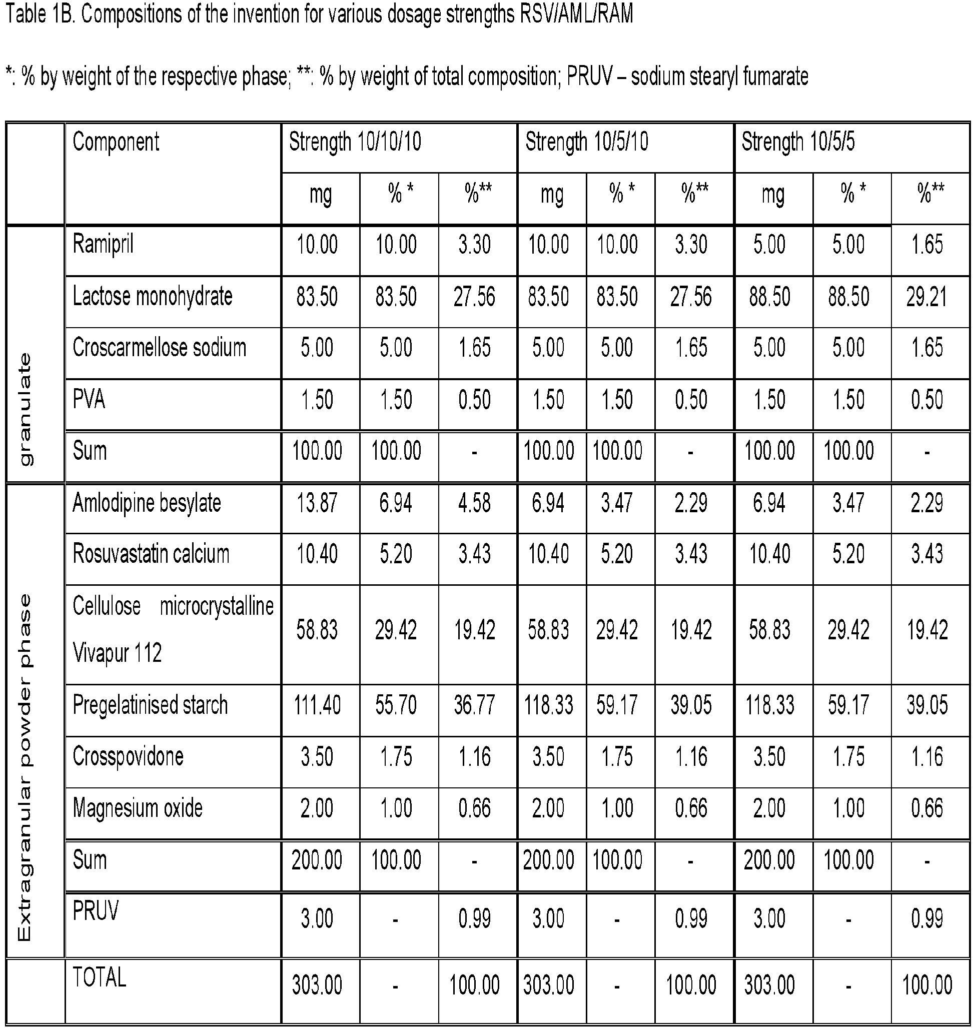

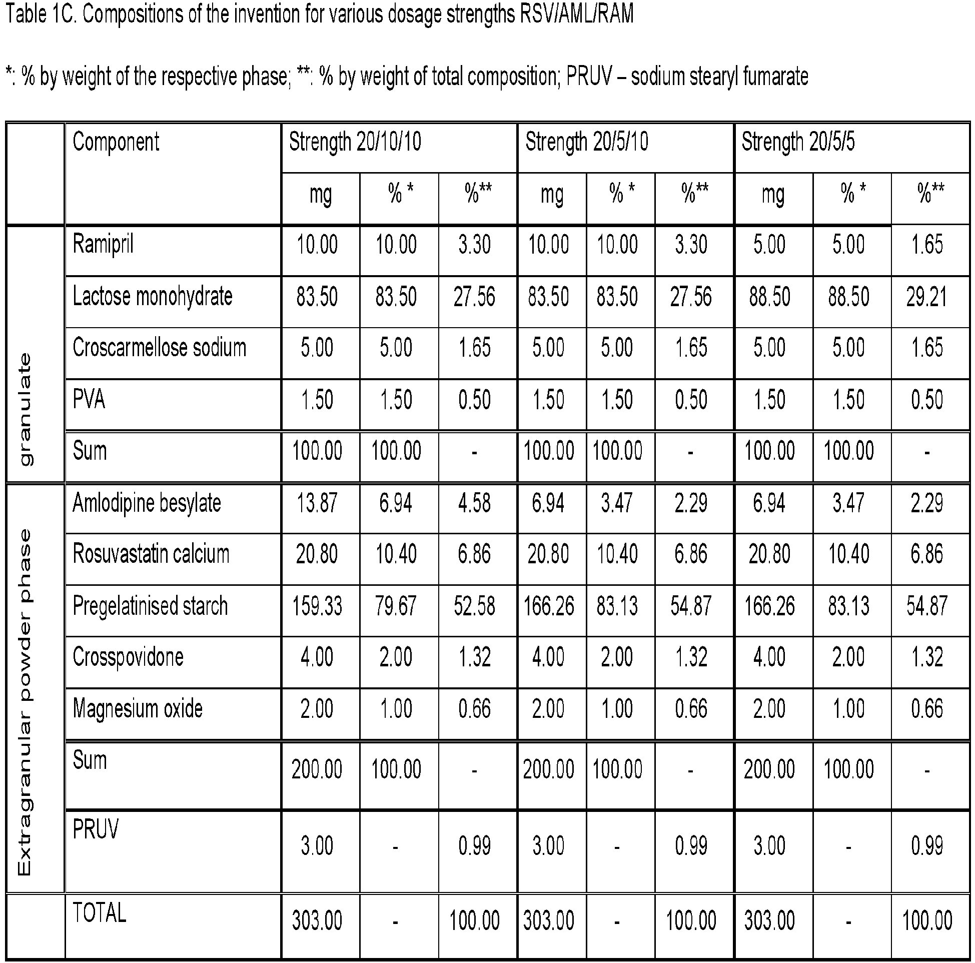

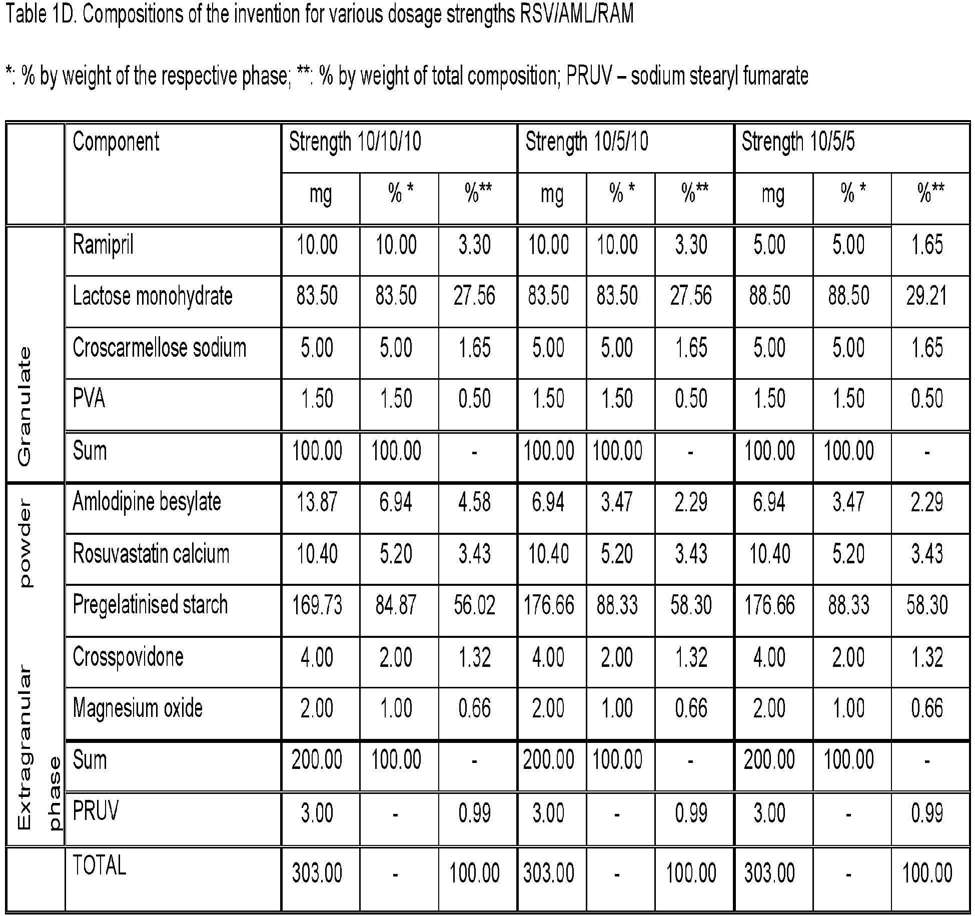

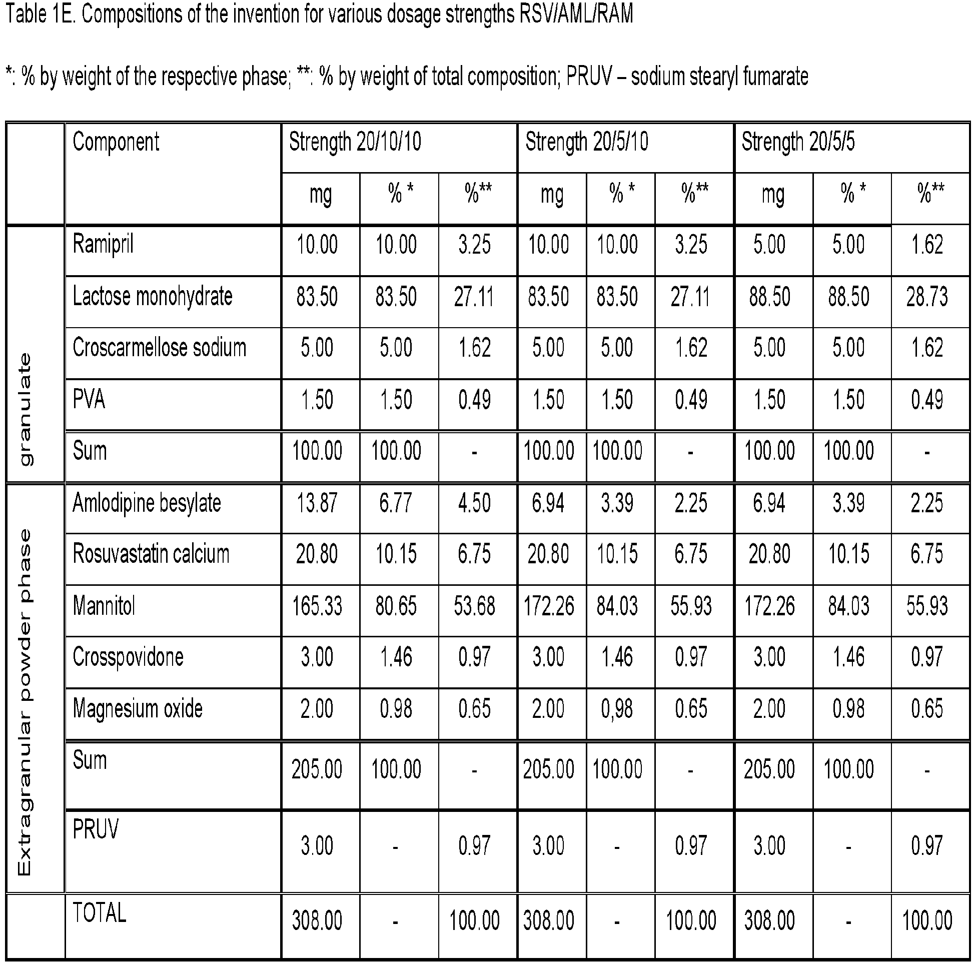

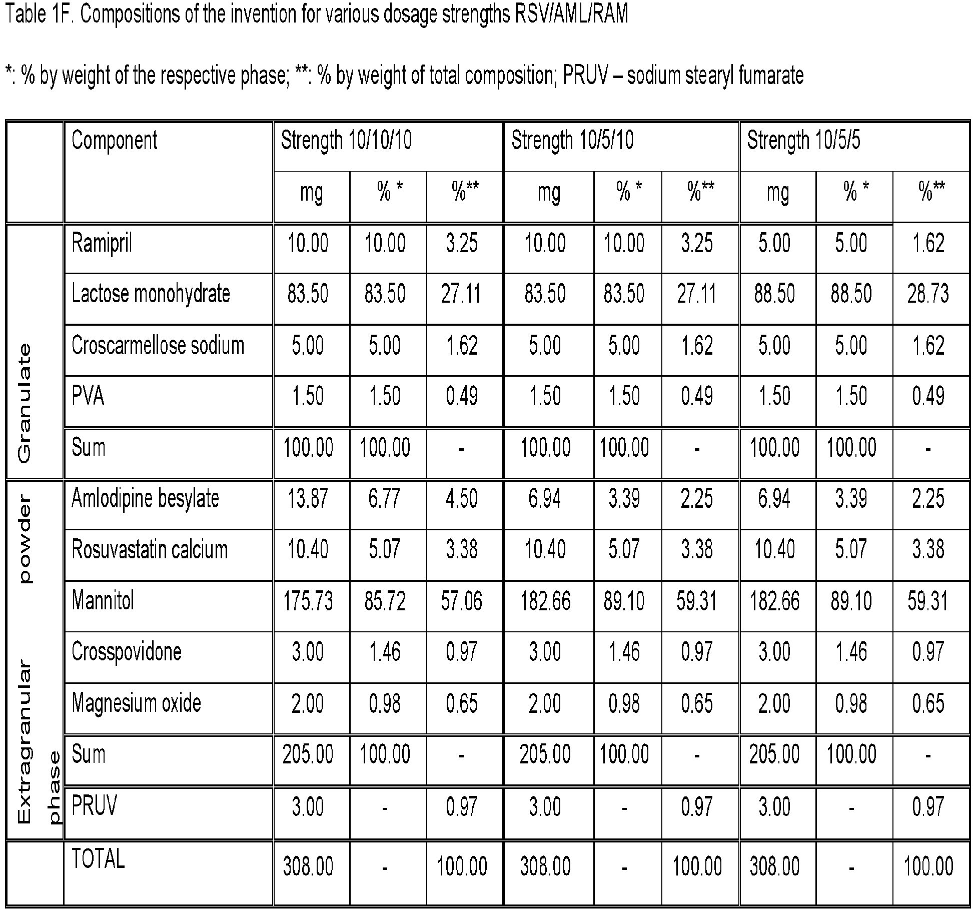

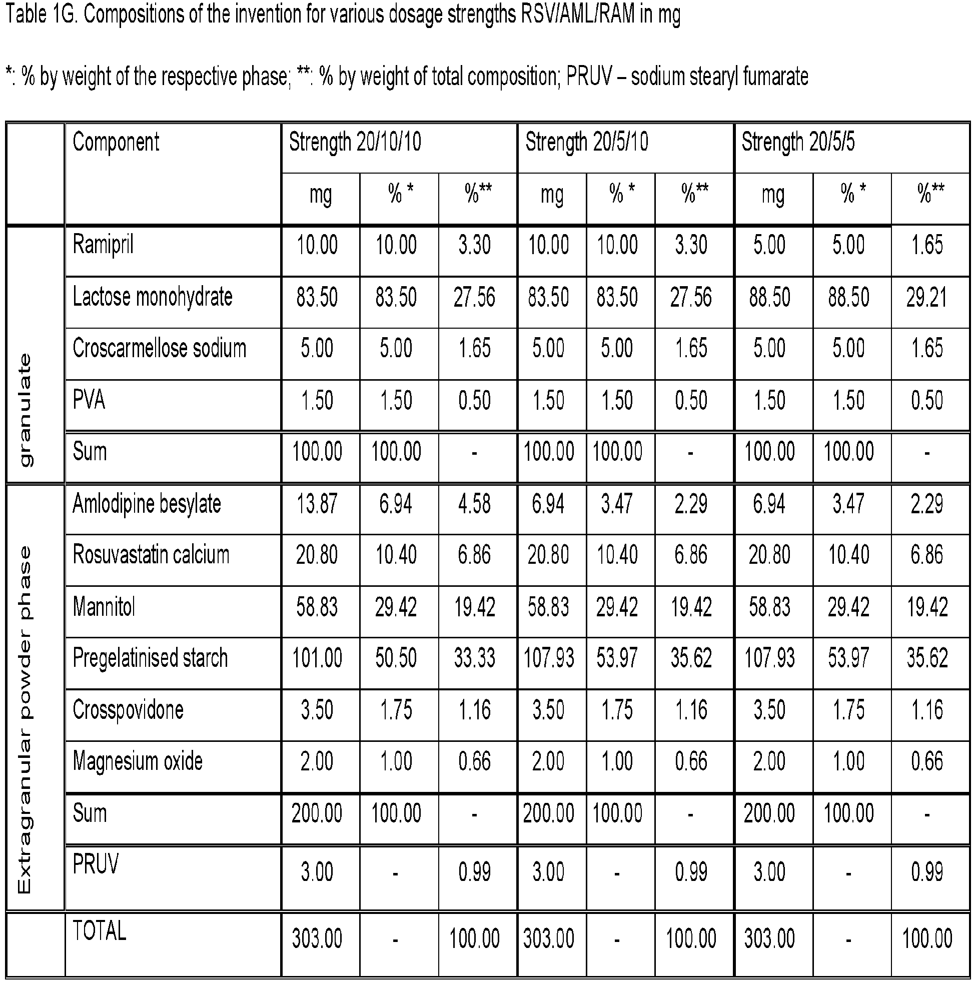

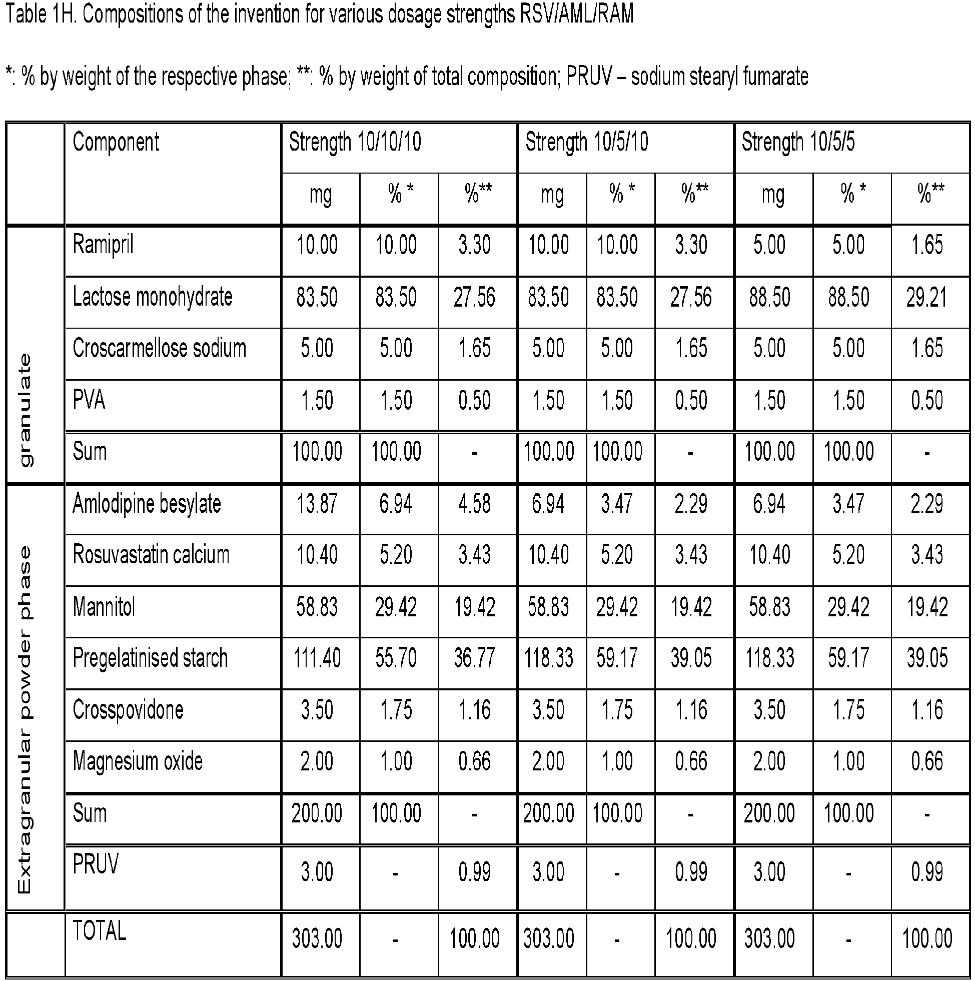

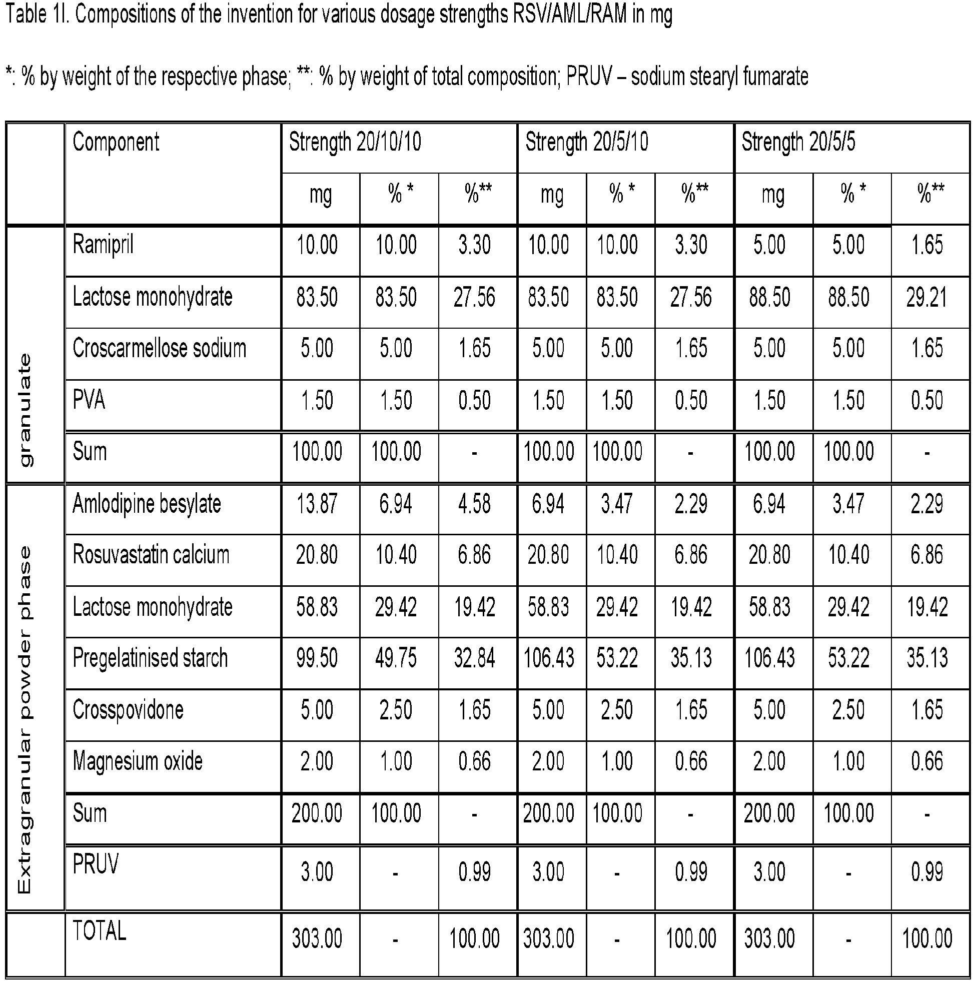

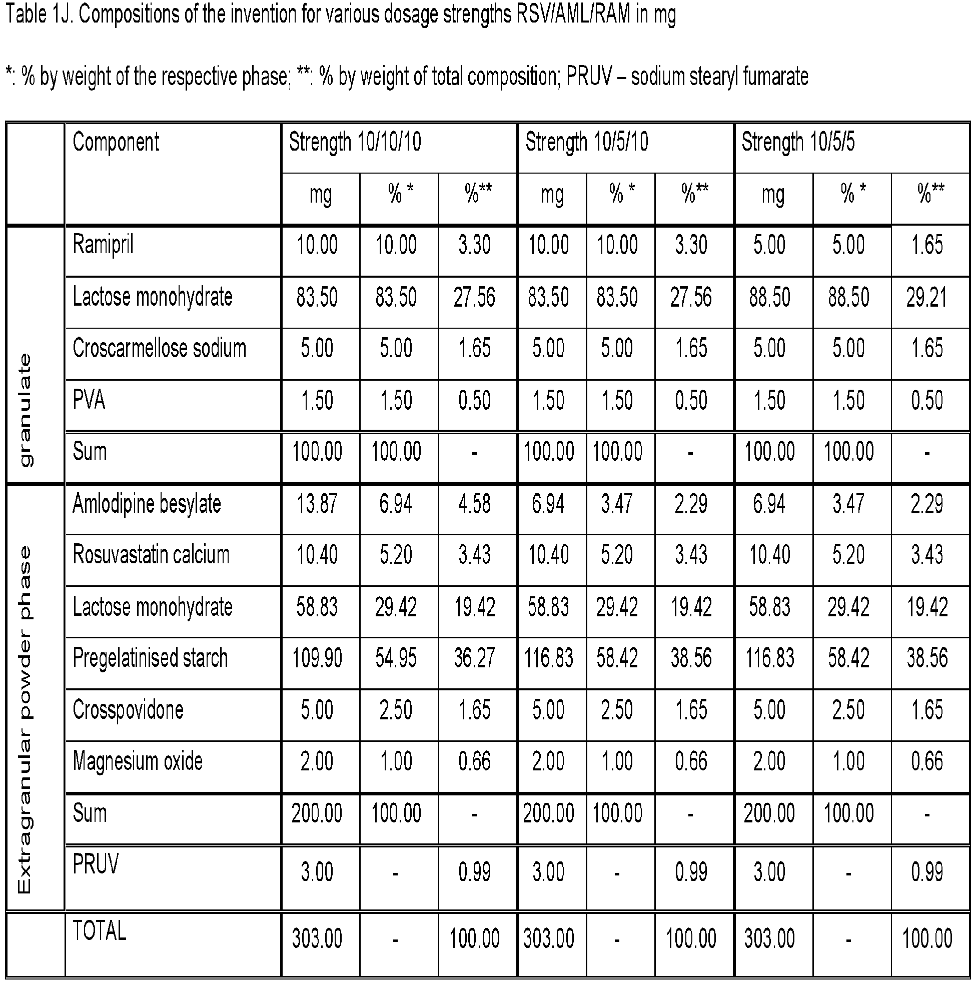

### Example 2. Testing stability and impurities contents of compositions of the invention.

Compositions of the invention (RAR25CA and RAR25CB) and comparative composition (RAR25C) at the dosage strengths 20/10/10 presented in Table 2 below were prepared as described in Example 1 above.

Composition RAR25 is the blend of ramipril granulate with extragranular powder blend RSV/AML. Stability of such blend without magnesium oxide and with addition of magnesium oxide (1% or 2% by weight with respect to the weight of the powder blend, RAR25C A and RAR25C B, respectively) was tested.

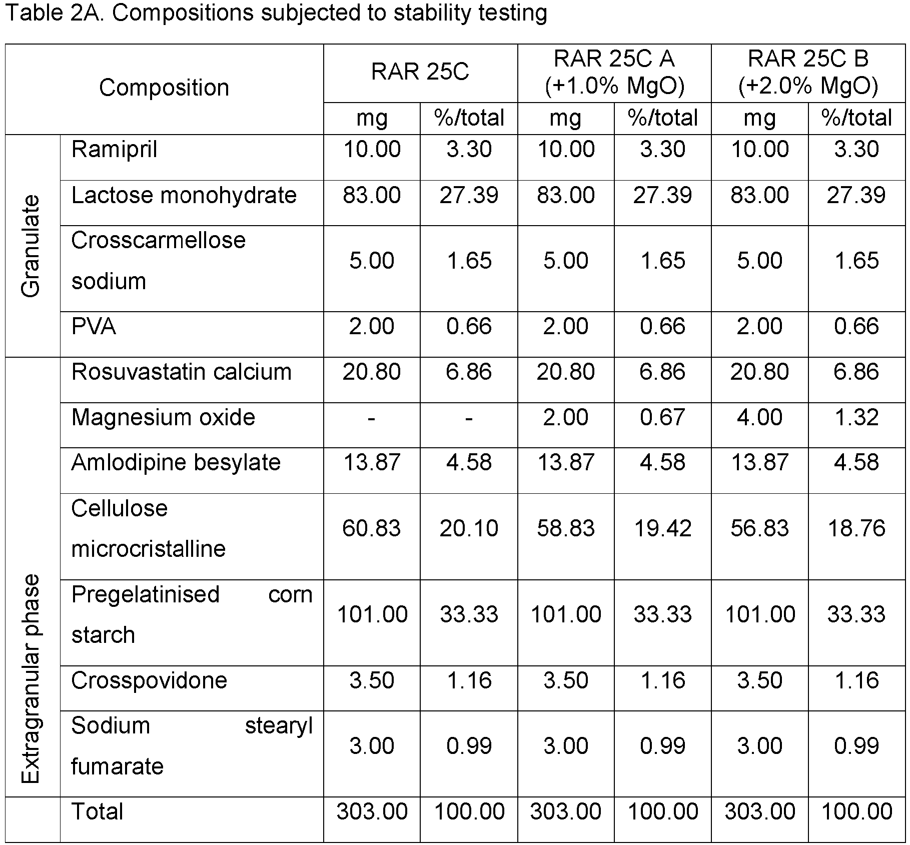

Stability tests of the RSV/AML/RAM combined compositions were carried out in the accelerated storage conditions 40°C/75%RH. Impurities contents in the compositions of the invention were measured and analyzed by HPLC method.

Results of the stability tests are presented in Table 2B below.

In Table 2B:
D-AML = 3-ethyl 5-methyl 2-[(2-aminoethoxy)methyl]-4-(2-chlorophenyl)-6-methyl-pyridine-3,5-dicarboxylate;
E-RAM = (2S,3aS,6aS)-1-[(2S)-2-[[(1S)-1-carboxy-3-phenylpropyl]amino]-propanoyl]octahydrocyclopenta[b]pyrrole-2-carboxylic acid (ramiprilat);
D-RAM = ethyl (2S)-2-[(3S,5aS,8aS,9aS)-3-methyl-1,4-dioxodecahydro-2H-cyclopenta[4,5]pyrrolo[1,2-a]pyrazin-2-yl]-4-phenylbutanoate (ramipril diketopiperazine);
B-RSV = (3RS,5RS,6E)-7-[4-(4-fluorophenyl)-2-(N-methylmethanesulfonamido)-6-(propan-2-yl)pyrimidin-5-yl]-3,5-dihydroxyhept-6-enoic acid;
C-RSV = (3R,6E)-7-[4-(4-fluorophenyl)-2-(N-methylmethanesulfonamido)-6-(propan-2-yl)pyrimidin-5-yl]-3-hydroxy-5-oxohept-6-enoic acid;
D-RSV = N-[4-(4-fluorophenyl)-5-[(1E)-2-[(2S,4R)-4-hydroxy-6-oxooxan-2-yl]ethen-1-yl]-6-(propan-2-yl)pyrimidin-2-yl]-N-methylmethanesulfonamide

It can be seen that addition of magnesium oxide lowers the amount of impurities, in particular D-RAM and D-RSV. Content of D-AML impurity is also low. Quality of the combined product of the invention is more than satisfactory after 6 months storage in accelerated conditions (40°C, 75%RH) (total impurity < 5.0% limit according to ICH Q1A (R2) guidelines). Especially important is decrease of impurity D-RSV, since its limit according to ICH Q1A (R2) guidelines is 0.5%. Without incorporation of magnesium oxide this regulatory limit is exceeded, the amount reaching 0.58 after 6 months, while incorporation of magnesium oxide allows to stay below regulatory limit, the amount being 0.34 after 6 months.

Apart from the fact that the compositions of the inventions are stable, other main specification requirements are fulfilled.

In particular, release profiles of all three active ingredients from the combined composition tested using basket method under the following conditions:
1) 0.1M HCl, 500 and 900 ml, 100 rpm
2) 0.01M HCl, 900 ml, 100 rpm
3) Acetate buffer, pH 4.5, 500 and 900 ml, 100 rpm
4) Phosphate buffer, pH 6.8, 500 and 900 ml, 100 rpm
complied with release profiles of respective reference mono-products, i.e. Crestor for rosuvastatin, Norvasc for amlodipine, and Tritace for ramipril. All three active ingredients were releases in more than 85% after 15 minutes. Surprisingly, the addition of magnesium oxide did not affect release rate.

Furthermore, the problem of uniformity of the contents of rosuvastatin and amlodipine is minimized or avoided due to the use of its powder mixtures without granulation.

**Table 2A. Results of the stability tests under accelerated conditions.**

| **Series** | **RAR25C** | | | | | | **RAR25C A (1% MgO with respect to the extragranular phase)** | | | | | |
|---|---|---|---|---|---|---|---|---|---|---|---|---|
| **Strength RSV/AML/RSV** | **20/10/10** | | | | | | **20/10/10** | | | | | |
| Conditions | 40°C 75%RH | | | | | | 40°C 75%RH | | | | | |
| Time point | start | 2W | 1M | 2M | 3M | 6M | start | 2W | 1M | 2M | 3M | 6M |
| D-AML | 0.02 | 0.02 | 0.02 | 0.02 | 0.02 | 0.02 | 0.02 | 0.02 | 0.02 | 0.02 | 0.02 | 0.02 |
| E-RAM | | | | | | 0.06 | | | | | | 0.08 |
| D-RAM | 0.13 | 0.23 | 0.47 | 0.89 | 1.09 | **1.84** | 0.13 | 0.23 | 0.39 | 0.66 | 0.77 | **1.16** |
| B-RSV | 0.08 | 0.08 | 0.07 | 0.08 | 0.07 | 0.08 | 0.08 | 0.08 | 0.08 | 0.08 | 0.07 | 0.08 |
| C-RSV | 0.03 | 0.06 | 0.11 | 0.16 | 0.,18 | 0.30 | 0.03 | 0.06 | 0.10 | 0.15 | 0.17 | 0.26 |
| D-RSV | 0.00 | 0.02 | 0.12 | 0.22 | 0.32 | **0.58** | 0.01 | 0.02 | 0.08 | 0.15 | 0.22 | **0.34** |
| RRT 0.69 | 0.04 | 0.04 | 0.04 | 0.04 | 0.04 | 0.03 | 0.04 | 0.04 | 0.08 | 0.04 | 0.04 | 0.04 |
| RRT 0.95 | 0.02 | 0.20 | 0.01 | 0.03 | 0.02 | 0.03 | 0.02 | 0.02 | 0.03 | 0.03 | 0.02 | 0.01 |
| RRT 1.22 | 0.04 | 0.03 | 0.04 | 0.05 | 0.04 | 0.06 | 0.04 | 0.03 | 0.04 | 0.05 | 0.04 | 0.05 |
| **Sum** | **0.37** | **0.50** | **0.88** | **1.49** | **1.79** | **3.00** | **0.38** | **0.11** | **0.81** | **1.18** | **1.35** | **2.06** |

| | | | | | | | | | | | | |
|---|---|---|---|---|---|---|---|---|---|---|---|---|
| RRT- relative retention time; RH- relative humidity; W-weeks; M - months | | | | | | | | | | | | |

## Claims

1. A fixed-dose solid pharmaceutical composition, comprising ramipril, amlodipine or its pharmaceutically acceptable salt, and rosuvastatin or its pharmaceutically acceptable salt, said composition comprising or consisting of a blend of:
a) a ramipril granulate comprising or consisting of ramipril in the intimate mixture with intragranular polyvinyl alcohol as a binder, a filler, and a disintegrant, the weight ratio of ramipril to polyvinyl alcohol being in the range from 1.1 to 10.0;
b) an extragranular phase comprising or consisting of:
- b1) a powder blend of amlodipine or its pharmaceutically acceptable salt, rosuvastatin or its pharmaceutically acceptable salt, one or more fillers, a disintegrant, and an alkaline-environment producing organic or inorganic stabilizing agent, and
- b2) a glidant.

2. The composition of claim 1, wherein the alkaline-environment producing organic or inorganic stabilizing agent in powder blend b1) is magnesium oxide.

3. The composition of claim 1 or 2, wherein the weight ratio of ramipril to polyvinyl alcohol in the ramipril granulate is in the range of 2.0 to 7.0, preferably about 3.3 or 6.7.

4. The composition of any one of claims 1 to 3, wherein in the ramipril granulate a) the filler is selected from the group consisting of lactose, cellulose and its derivatives, such as microcrystalline cellulose, sugars and sugar alcohols; and inorganic fillers such as calcium hydrogen phosphate, and preferably is lactose monohydrate.

5. The composition of any one of claims 1 to 4, wherein in the ramipril granulate a) the disintegrant is selected from the group consisting of calcium or sodium carboxymethylcellulose (sodium croscarmellose, CMC-Ca, CMC-Na); cross-linked polyvinylpyrrolidone (crosspovidone) type A or type B; and sodium starch glycolate, and preferably is crosscarmellose sodium.

6. The composition of any one of claims 1 to 5, wherein the filler in the powder blend b1) is selected from the group consisting of microcrystalline cellulose, pregelatinised starch, mannitol, lactose monohydrate, and their mixtures.

7. The composition of claim 6, wherein the filler in the powder blend b1) is the mixture of pregelatinised starch and microcrystalline cellulose.

8. The composition of any one of claims 1 to 7, wherein the disintegrant in the powder blend b1) is crosspovidone.

9. The composition of any one of claims 1 to 8, wherein the glidant b2) is sodium stearyl fumarate.

10. The composition of any one of claims 1 to 9, wherein the ramipril granulate is prepared by wet-granulation.

11. The composition of any one of claims 1 to 10, wherein pH of the aqueous solution of 50 mg of the ramipril granulate sample with the concentration of 0.31 mg of Ramipril/ 1 mL of water, measured at 20°C, is in the range from 5.0 to 6.0.

12. The composition of any one of claims 1 to 11, wherein amlodipine pharmaceutically acceptable salt is amlodipine besylate.

13. The composition of any one of claims 1 to 12, wherein rosuvastatin pharmaceutically acceptable salt is rosuvastatin calcium.

14. A process for the preparation of the fixed-dose solid pharmaceutical composition as defined in any one of claims 1 to 13, the process comprising:
- preparation of the ramipril granulate a) by wet granulation of a blend comprising ramipril, a filler, and disintegrant using a solution of polyvinyl alcohol as a granulation liquid, preferably aqueous solution of polyvinyl alcohol;
- preparation of the powder blend b1) comprising amlodipine or its pharmaceutically acceptable salt, rosuvastatin or its pharmaceutically acceptable salt, an alkaline-environment producing organic or inorganic stabilizing agent, a filler, and optionally one or more further excipients;
- blending ramipril granulate a) with the powder blend b1);
- adding the glidant b2) and homogenizing.

15. The process of claim 14, wherein the powder blend b) is performed by preparing amlodipine premix with the filler or part thereof, preparing rosuvastatin premix with the alkaline-environment producing organic or inorganic stabilizing agent and the filler or part thereof, combining both premixes with remaining part of the fillers and disintegrant and mixing.

16. The process of claim 14 or 15, further comprising filling the solid composition into capsules.

17. A pharmaceutical unit dosage form, comprising a capsule filled with the pharmaceutical composition as defined in any one of claims 1 to 13 or prepared as defined in any one of the claims 14 or 15.
